# EUROPEAN PATENT SPECIFICATION

(11) **EP 1 730 499 B1**
(45) Date of publication and mention of the grant of the patent: **26.08.2009**
(21) Application number: 05718286.7
(22) Date of filing: 23.03.2005
(51) Int. Cl.: G01N 21/64, G01N 33/52, C07K 14/435

(54) **METHOD FOR THE DETECTION OF INTRACELLULAR PARAMETERS WITH LUMINESCENT PROTEIN PROBES FOR THE SCREENING OF MOLECULES CAPABLE OF ALTERING SAID PARAMETERS**
VERFAHREN ZUR DETEKTION VON INTRAZELLULÄREN PARAMETERN MIT LUMINESZIERENDEN PROTEINSONDEN ZUR SELEKTION VON MOLEKÜLEN, DIE DIESE PARAMETER ÄNDERN KÖNNEN
PROCEDE DE DETECTION DE PARAMETRES INTRACELLULAIRES AU MOYEN DE SONDES PROTEIQUES LUMINESCENTES POUR LE CRIBLAGE DE MOLECULES CAPABLES DE MODIFIER LESDITS PARAMETRES

(30) Priority: 26.03.2004 IT MI20040598
(43) Date of publication of application: 13.12.2006
(73) Proprietor: Aequotech S.r.l., 44100 Ferrara (IT)
(72) Inventor: RIZZUTO, Rosario, I-35137 Padova (IT); POZZAN, Tullio, I-30031 Dolvo (IT); PINTON, Paolo, I-35132 Padova (IT); GIORGI, Carlotta, I-46037 Roncoferraro (IT)
(74) Representative: Iannone, Carlo Luigi
(86) International application number: PCT/IB2005/000795
(87) International publication number: WO 2005/093429

(56) References cited:
- WO-A-00/02045
- WO-A-03/082904
- US-B1- 6 566 083
- DUPRIEZ V J ET AL: "AEQUORIN-BASED FUNCTIONAL ASSAYS FOR G-PROTEIN-COUPLED RECEPTORS, ION CHANNELS, AND TYROSINE KINASE RECEPTORS" RECEPTORS AND CHANNELS, HARWOOD ACADEMIC PUBLISHERS, CH, vol. 8, no. 5/6, 2002, pages 319-330, XP009055298 ISSN: 1060-6823
- DUPRIEZ V J: "Calcium in drug screening" CALCIUM: THE MOLECULAR BASIS OF CALCIUM ACTION IN BIOLOGY AND MEDICINE, X, XX, 2000, pages 647-659, XP009089634
- MARSAULT R ET AL: "DOMAINS OF HIGH CA2+ BENEATH THE PLASMA MEMBRANE OF LIVING A7R5 CELLS" EMBO JOURNAL, OXFORD UNIVERSITY PRESS, SURREY, GB, vol. 16, no. 7, 1997, pages 1575-1581, XP009055307 ISSN: 0261-4189
- POZZAN T ET AL: "INVESTIGATING SIGNAL TRANSDUCTION WITH GENETICALLY ENCODED FLUORESCENT PROBES" EUROPEAN JOURNAL OF BIOCHEMISTRY, BERLIN, DE, vol. 270, no. 11, June 2003 (2003-06), pages 2343-2352, XP009055300 ISSN: 0014-2956
- SATO KEN-ICHI ET AL: "Adaptor protein Shc is an isoform-specific direct activator of the tyrosine kinase c-Src" JOURNAL OF BIOLOGICAL CHEMISTRY, AMERICAN SOCIETY OF BIOLOCHEMICAL BIOLOGISTS, BIRMINGHAM,, US, vol. 277, no. 33, 4 June 2002 (2002-06-04), pages 29568-29576, XP002403514 ISSN: 0021-9258
- PINTON P ET AL: "DYNAMICS OF GLUCOSE-INDUCED MEMBRANE RECRUITMENT OF PROTEIN KINASE C BETAII IN LIVING PANCREATIC ISLET BETA-CELLS" JOURNAL OF BIOLOGICAL CHEMISTRY, AMERICAN SOCIETY OF BIOLOCHEMICAL BIOLOGISTS, BIRMINGHAM,, US, vol. 277, no. 40, 4 October 2002 (2002-10-04), pages 37702-37710, XP009055334 ISSN: 0021-9258
- OANCEA E: "Green fluorescent (GFP)-tagged Cysteine-rich domains from protein kinase C as fluorescent indicators for diacylglycerol signalling in living cells" THE JOURNAL OF CELL BIOLOGY, ROCKEFELLER UNIVERSITY PRESS, US, vol. 140, no. 3, 9 February 1998 (1998-02-09), pages 485-498, XP002111850 ISSN: 0021-9525
- TORFS HERBERT ET AL: "Recombinant aequorin as a reporter for receptor-mediated changes of intracellular Ca2+-levels in Drosophila S2 cells" INVERTEBRATE NEUROSCIENCE, vol. 4, no. 3, April 2002 (2002-04), pages 119-124, XP002348989 ISSN: 1354-2516
- LE POUL EMMANUEL ET AL: "Adaptation of aequorin functional assay to high throughput screening" JOURNAL OF BIOMOLECULAR SCREENING, vol. 7, no. 1, February 2002 (2002-02), pages 57-65, XP009055395 ISSN: 1087-0571
- BRINI M ET AL: "Nuclear targeting of aequorin: A new approach for measuring nuclear Ca-2+ concentration in intact cells" CELL CALCIUM, vol. 16, no. 4, 1994, pages 259-268, XP009055350 ISSN: 0143-4160
- CHIESA A ET AL: "RECOMBINANT AEQUORIN AND GREEN FLUORESCENT PROTEIN AS VALUABLE TOOLS IN THE STUDY OF CELL SIGNALLING" BIOCHEMICAL JOURNAL, THE BIOCHEMICAL SOCIETY, LONDON, GB, vol. 355, no. 1, 1 April 2001 (2001-04-01), pages 1-12, XP009013205 ISSN: 0264-6021

## Description

The present invention relates to a method for the detection of intracellular parameters by means of luminescent recombinant protein probes, for the screening of molecules capable of altering said parameters.

In particular, the present invention relates to a method for detecting intracellular parameters and/or activities by means of luminescent protein probes for the screening of molecules of pharmacological and/or cosmetic and/or environmental interest capable of altering said parameters and/or activities.

It is known that cells forming the organism have a continuous exchange of information, both with other cells and with the extracellular environment.

Communication between the cells is guaranteed by their capacity to receive and send signals of a chemical nature interacting with specific receptors present on their plasmatic membrane. In most cases, the activation of these receptors causes the generation of intracellular messengers, called second messengers, which convey the information brought by the extra-cellular messenger (or first messenger) into the cell. With respect to the extreme variety of extra-cellular mediators, the number of known second messengers is surprisingly limited: the calcium ion (Ca²⁺), inositol 1,4,5 triphosphate (IP₃), dia-cylglycerol (DAG), nucleotides of adenosine and cyclic nucleotides (cAMP and cGMP).

These second messengers in turn activate a series of intracellular performers, called cellular response effectors, which, through a variation in the intracellular parameters, are capable of triggering a series of events until a definitive cellular response is obtained.

Cellular parameters are those values, such as concentration, activation state, cellular localization, which enable us to understand and consequently describe the activity exerted by each single element present inside the cell (ions, proteins, nucleotides, signal molecules).

Variation of said cellular parameters refers to a modification in the normal basic indexes (variation in concentration, translocation to the plasmatic membrane, activation or inactivation), in response to certain stimuli (physiological or pharmacological) deriving from the extra-cellular environment.

Environmental, chemical, physical or genetic factors can create pathological conditions which cause alterations of the transduction pathways of the cellular signal.

In order to understand the functioning of a drug, it is necessary to avail of methods which allow the accurate monitoring of the various intracellular parameters on which the drugs and their variations must act.

At present, there are techniques for detecting only some of the cellular mediators involved in the transduction pathways of the signal and in most cases, these techniques are not efficient and/or easily applicable to massive screening methods of thousands of molecules, a strongly felt requirement, on the other hand, on the part of pharmaceutical, chemical or cosmetic industries.

The most widely-studied cellular mediator was and still is the calcium ion (Ca²⁺) . It has been seen that a wide variety of stimuli, ranging from growth factors to neuro-transmitters, are transmitted inside the cell by means of variations in the cytosol concentration of Ca²⁺ ([Ca²⁺]_{c}).

Ca²⁺ is an ubiquitous ion normally present in all cellular compartments, which, through variations in its concentration, is capable of regulating numerous cellular functions.

The development of the first techniques for measuring the concentration of Ca²⁺ goes back to the sixties' and represented the start of a detailed understanding of the mechanisms that regulate [Ca²⁺]_{c} and its role in controlling the numerous biological functions.

The first [Ca²⁺]_{c} measurements were effected by micro-injecting into huge cells of *Balanus,* a Ca²⁺-sensitive photo-protein, aequorin (Ridgway and Ashley, 1967); in subsequent years, metallochromic indicators and specific micro-electrodes were often used. In both cases, the techniques were limited by the necessity of micro-injecting the indicator or inserting an electrode into the cell and consequently by the possibility of using few large dimensional cellular types.

In the nineties', the use of fluorescent Ca²⁺ indicators such as, for example, quin2 and fura-2, capable of passing through the plasmatic membrane and remaining entrapped at a cytoplasmatic level (Tsien et al., 1982, Grynkiewicz et al., 1985), allowed the cellular homeostasis of Ca²⁺ to be studied in many cellular types and to demonstrate the ubiquitous role of this ion. The facility of use of fluorescent indicators, however, is opposed by the incapacity of being selectively accumulated in the various cellular organelles.

The above techniques, however, cannot be easily applied to pharmacological screening studies and are in any case limited to the measuring of Ca²⁺ alone.

The arrival of molecular biological techniques contributed, in the nineties', to the development of new method for monitoring the Ca²⁺ ion based on the construction of protein probes which are introduced inside the cells by means of transfection techniques. There are currently two categories of recombinant probes for Ca²⁺.

The first group exploits the fluorescent characteristics of molecules deriving from the fluorescent protein GFP (Green Fluorescent Protein, Zhang et al., 2002). These probes are difficult to use for pharmacological screening as they require extremely costly procedures in terms of time and the ratio between the fluorescent signal obtained under standby and activation conditions is not sufficiently wide to allow use for automated methods.

The second category of probes, is based on the use of bioluminescent proteins capable of binding the Ca²⁺ and consequently emitting a luminous radiation, which can be measured and correlated to the concentration of the Ca²⁺ itself, among which aequorin.

Aequorin, extracted and purified in 1962 from a species of luminescent medusa, *Aequorea Victoria* (Shimomura et al., 1962), is a protein of about 22 kDa consisting of an apoprotein and a hydrophobic prosthetic group, celentherazine, bound to the apoprotein by means of a covalent link of the peroxide type.

The Ca²⁺ link with aequorin at a level of 3 specific high affinity sites of the "EF-hand" type, induces a conformational modification of the protein itself and consequent breakage of the covalent link and emission of photons and release of the oxidized coenzyme.

It is possible to measure the variation in the [Ca²⁺] thanks to the existence of a relation between the logarithm of the emission rate of photons (L), expressed as a fraction with respect to the maximum luminescence rate, i.e. under saturation conditions (L/Lₘₐₓ) and the logarithm of [Ca²⁺]. These values, obtained through in *vitro* measurements, under known conditions of pH, ion strength [Mg²⁺] and temperature, allow a sigmoid-shaped calibration curve to be constructed, on the basis of which, within the range of Ca²⁺ concentrations from 0.1-10 µM (and over 100 µM using mutated aequorin (Montero et al., 1995), it is possible to correlate, at each moment, the fraction of aequorin consumed with the [Ca²⁺] value at which the photo-protein is exposed. The conversion of the luminous signal obtained from the cells, is based on this relation, in values of [Ca²⁺].

Aequorin, as bioluminescent marker, has advantages with respect to fluorescent indicators as it can be easily directed into the various cellular compartments by the use of appropriate regulating elements or signal peptides, representing a reliable instrument for measuring the variations in the concentration of Ca²⁺ (Rizzuto et al., 1992). In particular, this publication describes the technique for directing and measuring the Ca²⁺ concentrations at a mitochondria level.

Various recombinant probes of aequorin were subsequently developed, directed towards other cellular compartments such as, for example, nucleus (Brini et al., 1993; Brini et al., 1994), endoplasmatic reticulum (Montero et al., 1995), Golgi apparatus (Pinton et al., 1998), mitochondria inter-membrane space (Rizzuto et al., 1998), sarcoplasmatic reticulum (Brini et al., 1997), sub-membrane region (Marsault et al., 1997) which made it possible to determine the variations in the concentration of Ca²⁺ in the various cellular districts following various kinds of stimulation.

Another advantage of luminescence is that, with respect to fluorescence, it does not require an excitation light thus avoiding self-fluorescence and photobleaching phenomena. Furthermore, aequorin is not toxic, it does not form links with other cations and does not interfere with the intracellular Ca²⁺ concentration.

The authors previously studied the use of a Ca²⁺ sensitive recombinant photo-protein, such as aequorin, expressed in mammal cells as an alternative method for measuring the concentration of the Ca²⁺ ion (Rizzuto et al., 1993; Rizzuto et al., 1994; Brini et al., 1994b; Rizzuto et al., 1995; Brini et al., 1995; De Giorgi et al., 1996; Rutter et al., 1996; Brini et al., 1999; Robert et al., 2000; Porcelli et al., 2001; Chiesa et al., 2001).

The use of aequorin has a series of technique advantages described hereunder:
a) optimum signal/noise relation as mammal cells do not have endogenous luminescent protein;
b) it integrates the data of a transfected population, thus avoiding errors depending on cellular variability;
c) the aequorin probe can be stably expressed alone in a cellular line (thus obtaining a "permanent" and repeatable screening system), or stably co-expressed with specific receptors of interest (thus simplifying the functional analysis of the specific signaling systems);
d) extremely simple detection system, as it is sufficient to collect the whole light emission spectrum emitted, which is suitable for the miniaturization of the sample to be analyzed and automation, as the luminous signal is strong (the light emission increases logarithmically with the variation in the Ca²⁺ concentration).

It is interesting to observe, however, that although variations in the calcium ion can be easily detected, they are only involved in a few of the numerous signal transduction pathways of pharmaceutical interest. As already specified, there are, however, many other transduction pathways which involve other cellular elements (cellular effectors, enzymes, ion channels, second messengers) which are difficult to detect directly, but which are extremely important for identifying molecules of potential pharmacological or toxicological interest.

In view of what is indicated above, there is evidently the necessity for availing of effective, sensitive and rapid methods for the detection of intracellular activities, for the screening of molecules suitable for generating specific alterations in said activities.

International patent application WO-03/082,904 discloses modified aequorins and the use thereof in screening methods for the identification of agents that are capable of generating the alteration of a target intracellular event.

Chiesa et al. (2001) reviews the use of recombinant aequorin and green fluorescent proteins as tools to study cell signaling.

Marsault et al. (1997) uses a chimeric SNAP-25-aequorin to determine the Ca²⁺ concentration near the plasma membrane. The Ca²⁺ concentrations near the plasma membrane are >10-fold higher than those of the bulk cytosol.

Oancea et al. (1998) discloses that a chimeric recombinant aequorin comprising the first Cys-domain of PKC-y translocates form the cytosol to the plasma membrane upon stimulation of G-protein or tyrosine kinase coupled receptors.

The authors of the present invention have now developed new methods capable of indirectly detecting variations in the intracellular parameters, retracing said variations to corresponding variation of the concentration of the Ca²⁺ ion which can be easily detected. In particular, bioluminescent detections systems have been set up, based on the use of the aequorin photo-protein, capable of emitting luminous signals in response to variations in the concentration of Ca²⁺, which reflect the variation in enzymatic activities in response to certain physiological and/or pharmacological stimulations.

The conversion of intracellular parameters and activities into a variation of the calcium concentration enables the translocation of performer proteins, such as, for example, the kinase proteins (regulating proteins such as PKC) or the proteins of the shc family (protein adaptors such as p46shc, p52shc or p66shc) to be followed.

Among performer proteins of interest, the authors of the present invention examined, for illustrative purposes, the protein kinase C (PKC) and the protein p66shc. As PKC and p66shc, following activation, translocate from the cytoplasm to the plasmatic membrane where the concentration of the calcium ion is greater with respect to the concentration in the cytoplasm, the authors created a PKC/aequorin and p66shc/aequorin chimeric proteins particularly sensitive to the different Ca²⁺ concentration. In this way, the translocation to the membrane and consequent activation of the PKC and p66shc were correlated to an indirect parameter such as the different Ca²⁺ concentration in the two cellular areas (cytoplasm and sub-membrane area).

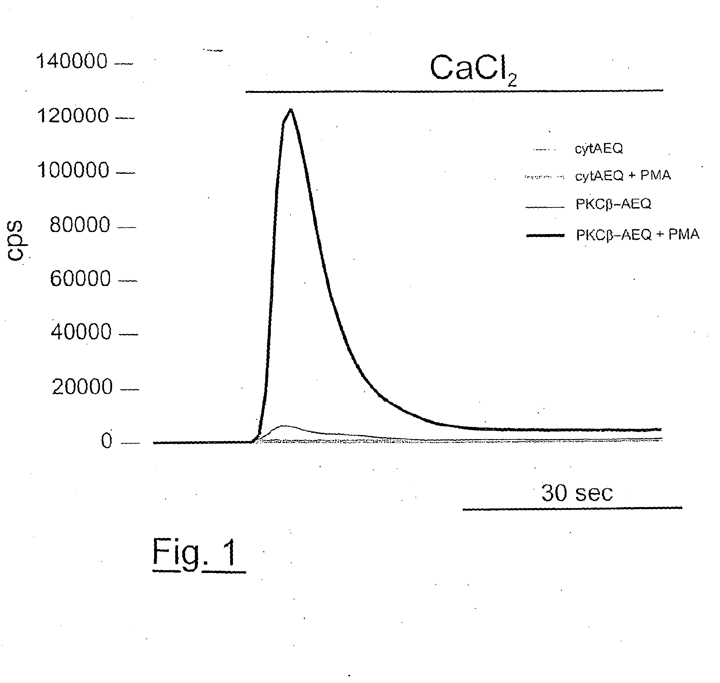

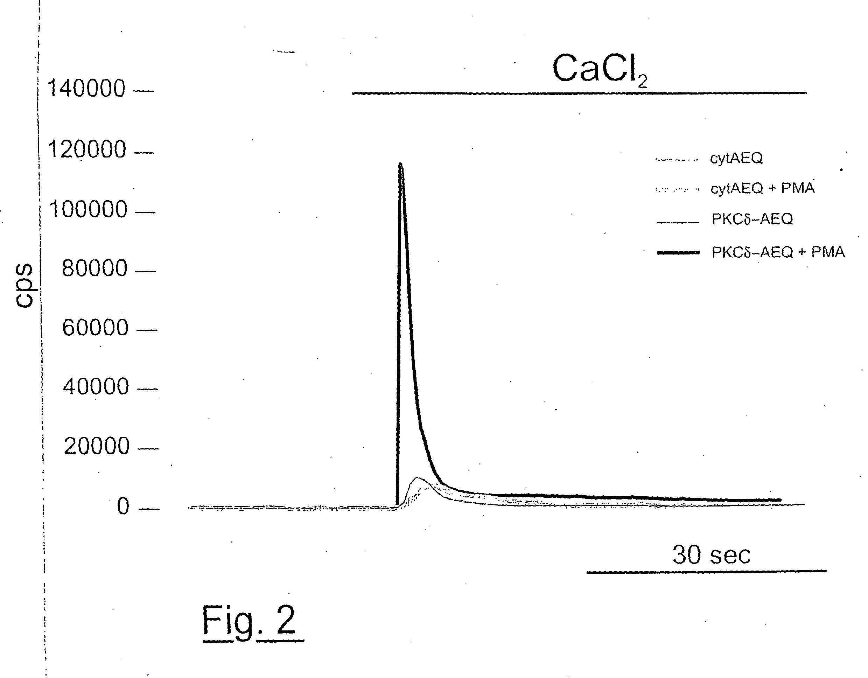

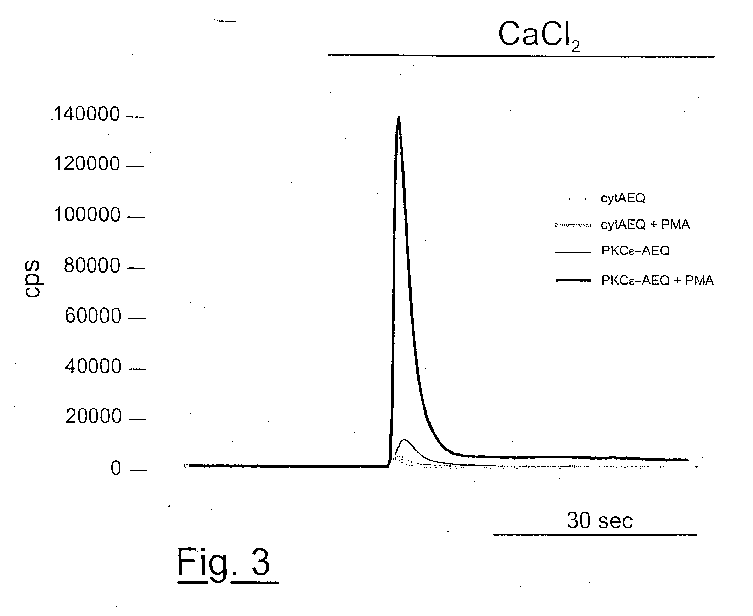

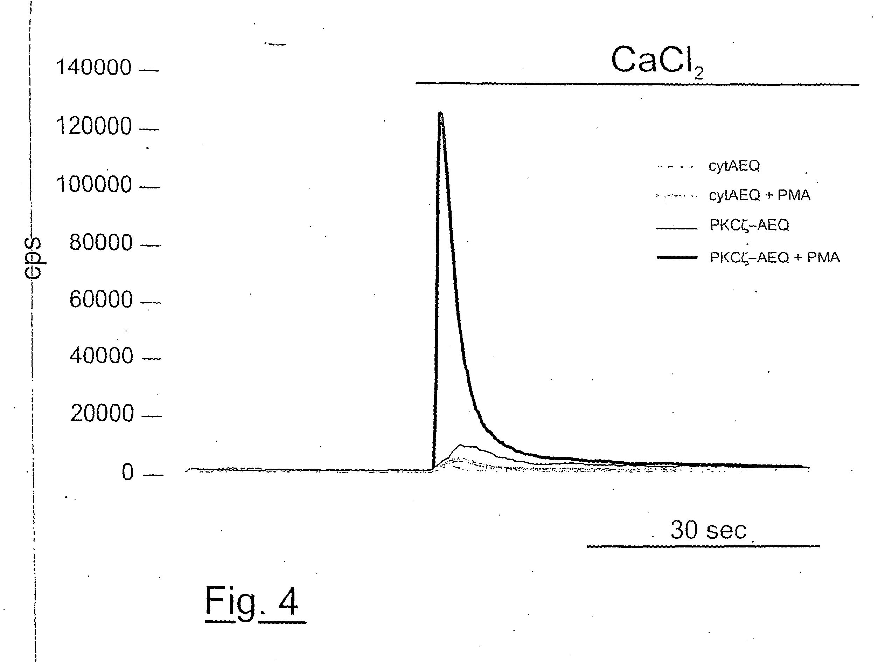

The authors show that the luminous signal of PKC-aequorin and p66shc-aequorin at the level of the membrane is (due to the non-linearity of the luminescence function with respect to the [Ca²⁺]) at least 50-100 times more intense with respect to that of a PKC-aequorin and p66shc-aequorin probe at a non-activated cytoplasmatic level. The luminescent signal of chimerical proteins considered is much more intense after induction of translocation with a known activator, as is shown in figures 1-6 relating to the experiments effected with a prototype of these chimerical proteins.

The method identified by the authors allows the translocation of the proteins to be simply, economically and efficiently quantified, as the light emission increases proportionally with the translocation degree, and a screening of compounds, active on said proteins, to be effected.

The various applications of the luminescent probes produced by the authors of the present invention, allow the differentiated screenings to be effected, of the molecules to be tested and as they can be easily automated, they can be used both for HTS (high throughput screening) analyses and for MTS (medium throughput screening) or LTS (low throughput screening) measurements.

The object of the present invention therefore relates to a screening method according to the enclosed claims.

The present invention, provides a screening method for molecules capable of generating the alteration of a target intracellular parameter, said alteration being the translocation from cytoplasm to the membrane of a cellular effector, said translocation being correlated to the different intracellular concentration of the Ca²⁺ ion between cytoplasm and submembrane area, detected by means of a Ca²⁺-sensitive recombinant aequorin probe, comprising the following phases:
a) construction of an expression vector containing the fusion protein sequence encoding said probe, said sequence being characterized in that it comprises sequences encoding at least one Ca²⁺-sensitive recombinant aequorin encoding sequence, condensed together with at least one cellular effector portion coupled to translocation; wherein said cellular effector portion coupled to translocation is selected between protein-kinases and adaptors;
b) transfection of at least one mammalian cell line with said vector containing the Ca²⁺-sensitive recombinant protein probe;
c) activation of said Ca²⁺-sensitive photo-protein by the addition of a prosthetic group to the cellular line expressing said recombinant protein probe;
d) administration of the molecule to be tested to the cellular line expressing said recombinant protein probe;
e) detection of the emission of photons on the part of the Ca²⁺-sensitive photo-protein expressed in the cellular line and evaluate the amount of activation or inhibition exerted by the tested molecule, on the basis of a ratio between the cps value obtained and the maximum value of cps registered under conditions of maximum stimulation of the cellular line.

For greater clarity, the term "Ca²⁺-sensitive photo-protein" refers to any amino acidic sequence capable of emitting photons, following the bond with calcium ions; aequorin and obelin proteins are particularly important from an applicative point of view.

"Performer or effector proteins" refers to regulating proteins, proteins which link membrane receptors, proteins which link membrane channels, proteins which link membrane lipids.

"Regulating protein" refers to any amino acidic sequence capable of modifying the activity and/or structure of other cellular components, following its interaction with other signal molecules.

"Proteins that link membrane receptors" refer to those amino acidic sequences capable of interacting with receptors situated at the level of cellular membranes. Among these, it is possible to distinguish adaptors which allow the interaction between the activated receptor and a third protein, and modulators which directly interfere with the activity of the receptor.

The term "proteins that link membrane channels" indicate those amino acidic sequences capable of interacting with channels situated at the level of cellular membranes.

Furthermore, the term "proteins that link membrane lipids" are identified as those amino acidic sequences capable of interacting with lipids situated at the level of cellular membranes.

Finally, the term "cellular compartment" refers to any region inside the cell preferably delimited by cellular membranes such as: cytoplasm, area below the plasmatic membrane, nucleus, mitochondria, endo-sarcoplasmatic reticulum, Golgi apparatus, vesicles, lyso-endosomes.

The present invention is described hereunder for illustrative but non-limiting purposes, according to its preferred embodiments, with particular reference to the figures of the enclosed drawings, in which:
figure 1 shows a graph which illustrates the measuring of the translocation entity (cps) of the probe with the isoform PKCβ-aequorin in the presence or in the absence of PMA stimulation and the difference with respect to the aequorin cytosolic cyt-AEQ probe;
figure 2 shows the graph which illustrates the measuring of the translocation entity (cps) of the probe with the isoform PKCδ-aequorin in the presence or in the absence of PMA stimulation and the difference with respect to the aequorin cytosolic cyt-AEQ probe;
figure 3 shows the graph which illustrates the measuring of the translocation entity (cps) of the probe with the PKCε-aequorin isoform in the presence or in the absence of PMA stimulation and the difference with respect to the aequorin cytosolic cyt-AEQ probe;
figure 4 shows the graph which illustrates the measuring of the translocation entity (cps) of the probe with the PKCζ-aequorin isoform in the presence or in the absence of PMA stimulation and the difference with respect to the aequorin cytosolic cyt-AEQ probe;
figure 5 shows a graph which illustrates the measuring of the translocation entity (cps) of the PKCβ-AEQ probe under hyperglycaemia conditions in the presence or absence of a drug;
figure 6 shows the graph which illustrates the measuring of the translocation entity (cps) of the p66shc-AEQ probe in the presence or in the absence of EGF stimulation and the difference with respect to the aequorin cytosolic cyt-AEQ probe.

### EXAMPLE 1: Analysis of the translocation to the plasmatic membrane of a cellular effector of interest, the kinase protein C (PKC).

### MATERIALS AND METHODS

### construction of a PKC-aequorin chimeric probe

In order to validate our procedure, various types of a series of PKC-aequorin chimeric probes were designed, each containing a different PKC isoform:
1) PKC beta-aequorin (beta PKC: ref. M13975)
2) PKC delta-aequorin (delta PKC: ref. M18330);
3) PKC epsilon-aequorin (epsilon PKC: ref. AF028009);
4) PKC zeta-aequorin (zeta PKC: ref. M18332);
5) PKC gamma-aequorin;
6) PKC alpha-aequorin (alpha PKC: ref. M13973);
7) PKC lambda-aequorin;
8) PKC theta-aequorin (theta PKC: ref. L07032);
9) PKC eta-aequorin.

In particular, the nucleotide sequence of aequorin used for the construction of PKC-aequorin probes is the following:

ATG AAG CTT TAT GAT GTT CCT GAT TAT GCT AGC CTC AAA
CTT ACA TCA GAC TTC GAC AAC CCA AGA TGG ATT GGA CGA
CAC AAG CAT ATG TTC AAT TTC CTT GAT GTC AAC CAC AAT
GGA AAA ATC TCT CTT GAC GAG ATG GTC TAC AAG GCA TCT
GAT ATT GTC ATC AAT AAC CTT GGA GCA ACA CCT GAG CAA
GCC AAA CGA CAC AAA GAT GCT GTA GAA GCC TTC TTC GGA
GGA GCT GGA ATG AAA TAT GGT GTG GAA ACT GAT TGG CCT
GCA TAT ATT GAA GGA TGG AAA AAA TTG GCT ACT GAT GAA
TTG GAG AAA TAC GCC AAA AAC GAA CCA ACG CTC ATC CGT

ATA TGG GGT GAT GCT TTG TTT GAT ATC GTT GAC AAA GAT
CAA AAT GGA GCC ATT ACA CTG GAT GAA TGG AAA GCA TAC
ACC AAA GCT GCT GGT ATC ATC CAA TCA TCA GAA GAT TGC
GAG GAA ACA TTC AGA GTG TGC GAT ATT GAT GAA AGT GGA
CAA CTC GAT GTT GAT GAG ATG ACA AGA CAA CAT TTA GGA
TTT TGG TAC ACC ATG GAT CCT GCT TGC GAA AAG CTC TAC
GGT GGA GCT GTC CCC TAA.

In the specific example, the results presented relate to the use of the PKC beta-aequorin (PCKβ-AEQ), PKC delta-aequorin (PCKδ-AEQ), PKC epsilon-aequorin (PCKε-AEQ), PKC zeta-aequorin (PCKζ-AEQ) probes.

These probes were obtained by inserting, in a eukaryotic expression vector (pcDNA3; 5.4 kb), the sequence encoding the various PKC isoforms condensed in frame with the sequence encoding aequorin described above.

The localization of the chimeric probe was determined by signal peptides contained inside the native sequence of the protein whose intracellular path is to be followed, in this specific case PKC. The addition of the cDNA of aequorin to the C-terminal of the sequence encoding PKC does not alter either the orientation or the physiological behaviour of the protein being examined.

At this point, transfection is effected in different cellular lines of the chimeric probe obtained, such as HeLa, Cos 7 or Hek 293 cells.

### Engineering of the cellular line

In the example illustrated, it was not necessary for the cellular line used to express any other element to activate the cellular response. It is possible, however, depending on study requirements, to induce the cells to express a specific receptor for the molecule/drug to be tested in order to ensure an effective linkage degree, thus simplifying the functional analysis of the transduction systems.

### Cellular transfection

The cells cultivated on cellular culture flasks (75 cm²) were transfected with a vector containing the chimeric probe produced, using the most suitable transfection techniques for the cellular line in question. As the HeLa stabilized cellular line was used as experimental model, the calcium phosphate technique was adopted as transfection method which guarantees a high percentage of positive cells for this cellular line.

### Collection of cells expressing the chimeric probe

36 hours after transfection, the cells contained in the flask were detached from the bottom by trypsinization. The cellular suspension was then transferred to a Falcon tube (15 to 50 ml) and subjected to centrifugation at 1200 rpm at 20°C and finally the cellular precipitate was re-suspended in KRB (Krebs-Ringer modified buffer: 125 mM NaCl , 5 mM KCl, 1 mM Na₃PO₄, 1 mM MgSO₄ 5.5 mM glucose, 20 mM HEPES, pH 7.4, 37°C).

### Reconstitution of the aequorin photo-protein to active form

The prosthetic group celentherazine was added to the suspension of cells expressing the PKC-aequorin probe at a final concentration of 5 µM.

### Planting on multi-well plates

50 µl of suspension containing transfected cells (corresponding to about 100,000 cells) were planted in each well of the plate. The cells were left to adhere for a time varying from 1 to 2 hours keeping them in the dark, due to the photo-instability of celentherazine.

### Detection of the response

At the end of the incubation time, the various molecules to be tested were added to each well and the plate with the treated cells was then put in direct contact with a photo-multiplier which measures the emission of photons on the part of aequorin.

### RESULTS

This simple test can be used for testing compounds capable of modulating the translocation of PKC in live cells.

Figures 1-4 show the graphs which indicates the measuring of the translocation to cps of PKC beta, delta, epsilon and delta, according to the method described above.

The examples shown were carried out on two parallel batches of cells:
a) HeLa cells expressing the PKC-aequorin (PKC-AEQ) probe (dark lines).
   These cells were in turn subdivided into:
   - HeLa PKC-aequorin cells treated with PMA (1 µM, SIGMA) to imitate the action of a drug (thick dark line);
   - control HeLa PKC-aequorin cells non treated with PMA (thin dark line);
b) HeLa cells expressing the cytosol aequorin probe (cyt-AEQ) (not condensed to any protein) (light lines).

These cells were in turn subdivided into:
- HeLa cells with cytosol aequorin treated with PMA to imitate the action of a drug (thick light line);
- control HeLa cells with cytosol aequorin non treated with PMA (thin light line).

As can be observed from the trend of the graphs shown in figure 1-4, in the absence of external calcium, the emission of photons (expressed as cps: counts per second) has a very low intensity (lower than 100 cps), under all the conditions examined. Under such conditions, in fact, the concentration of calcium in the cytoplasm, where the cyt-AEQ probes (both in the absence and in the presence of PMA forbol ester) and PKC-AEQ probes (in the absence of PMA) are located, and below the plasmatic membrane, i.e. where the PKC-AEQ probe translocates follow-ing treatment with PMA, is very low (0.1 µM).

The addition of external calcium induces a significant flow of this ion through the channels situated on the plasmatic membrane with a consequent increase in the concentration of calcium in the region below the plasmatic membrane. Under these conditions, an enormous increase in the emission of photons is registered (> 100,000 cps) only in the cells expressing the PKC-AEQ probe, treated with PMA, indicating the complete translocation of the kinase.

In all the other groups of cells, there is no significant variation in the emission of photons (lower than 10,000 cps) on the part of aequorin.

From the graph, it is evident that all the cps values higher than those observed in the absence of the maximal activator (PMA) will indicate a complete translocation of the kinase. With this system, it is therefore possible not only to identify the molecules capable of inducing a translocation, but also to evaluate the amount of activation or inhibition exerted by the molecule tested, on the basis of a ratio between the cps value obtained and the maximum cps value registered under conditions of maximum stimulation of the cellular line.

The translocation efficiency can be determined by expressing it on the basis of a range between the cps value obtained under control conditions (0%) (without PMA) and that obtained following maximal translocation (100%) (after treatment with PMA), which can be described as follows:
- GOOD activator for values ranging from 70% to 100% with respect to the maximal activator;
- MEDIUM activator for values ranging from 40% to 70% with respect to the maximal activator;
- POOR activator for values ranging from 10% to 40% with respect to the maximal activator;
- NON-activator for values below 10% with respect to the maximal activator.

This result clearly shows the validity of the system proposed for measuring the translocation of proteins from the cytosol compartment to the plasmatic membrane in response to external stimulations.

### EXAMPLE 2: Analysis of the translocation to the plasmatic membrane of beta PKC under hyperglycaemia conditions.

### MATERIALS AND METHODS

### Construction of the chimeric PKC-aequorin probe

A beta-aequorin PKC probe, already described in example 1, was used, with which HUVEC endothelial cells were transfected.

### Engineering of the cellular line

In the example provided, it was not necessary for the cellular line used to previously express any other element to start the cellular response. It is possible, however, depending on the study requirements, to induce the cells to express a specific receptor for the molecule/drug to be tested in order to assure a sufficient linkage degree, thus simplifying the functional analysis of the transduction systems.

### Cellular transfection

The cells cultivated on cellular culture flasks (75 cm²) were transfected with a vector containing the chimeric probe produced, using the most suitable transfection techniques for the cellular line in question. Endothelial cells of stabilized umbilical cord veins, called HUVEC, were used as the experimental model, which were transfected with the PKC beta-aequorin probe, using li-pofectamine which guarantees a high percentage of positive cells for this cellular line.

### Collection of the cells expressing the chimeric probe

36 hours after transfection, the cells contained in the flask were detached from the bottom by trypsinization. The cellular suspension was then transferred to a Falcon tube (of 15 or 50 ml) and subjected to centrifugation at 1,200 rpm and at 20°C, and finally the cellular precipitate was re-suspended in KRB (Krebs-Ringer modified buffer: 125 mM NaCl, 5 mM KCl, 1 mM Na₃PO₄, 1 mM MgSO₄ 5.5 mM glucose, 20 mM HEPES, ph 7.4, 37°C).

### Reconstitution of the aequorin photo-protein to active form

The prosthetic group celentherazine at a final concentration of 5 µM was added to the cell suspension expressing the PKC beta-aequorin probe.

### Planting on multi-well plates

50 µl of suspension containing transfected cells (corresponding to about 100,000 cells) were planted in each well of the plate. The cells were left to adhere for a time varying from 1 to 2 hours, keeping them in the dark, due to the photo-instability of celentherazine.

### Detection of the response

At the end of the incubation time, the various molecules to be tested were added to each well and the plate with the treated cells was then put in direct contact with a photo-multiplier which measures the emission of photons on the part of aequorin.

### RESULTS

This simple test can therefore be used for testing compounds capable of modulating the PKC translocation into the live cells.

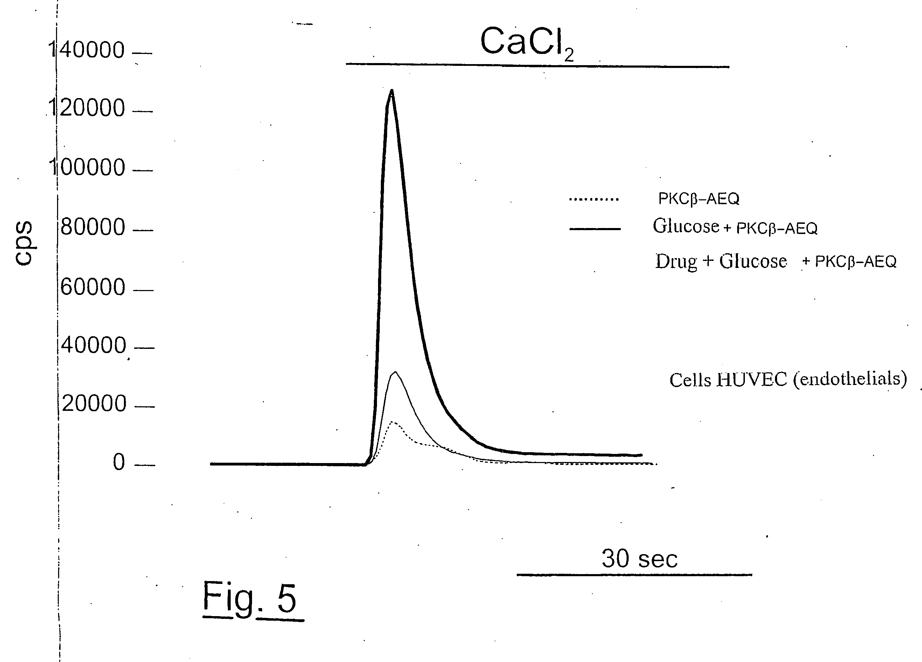

Figure 5 shows a graph which indicates the measuring of the PKC beta translocation, according to the method described above.

The example provided was carried out on three parallel batches of cells:
a) HUVEC cells expressing the PKC beta-aequorin (PKCβ-AEQ) probe, maintained under control conditions (absence of glucose)(dotted line).
b) HUVEC cells expressing the PKC beta-aequorin (PKCβ-AEQ) probe, maintained under hyperglycaemia conditions (10 mM of glucose)(dark, thick line).
c) HUVEC cells expressing the PKC beta-aequorin (PKCβ-AEQ) probe, maintained under hyperglycaemia conditions (10 mM of glucose) treated with a well-known anti-diabetic agent, metformin (20 µM) (dark, thin line).

As can be seen from the trend of the graph shown in figure 5, the emission of photons (expressed as cps: counts per seconds), in the absence of external calcium, is of a very low intensity (lower than 100 cps) in all conditions examined. In those conditions, in fact, the calcium concentration in the cytoplasm, where the PKCβ-AEQ probe (in the absence of glucose) is localized, and under the plasmatic membrane, i.e. where the PKCβ-AEQ probe translocates following treatment with glucose, is very low (0.1 µM).

The addition of external calcium induces a sustained flow of said ion through the channels situated on the plasmatic membrane with a consequent increase in the calcium concentration in the region under the plasmatic membrane. Under these conditions, an enormous increase in the emission of photons (> 100,000 cps) is registered only in the cells expressing the PKCβ-AEQ probe, treated with high glucose, indicating the completed translocation of kinase under hyperglycaemia conditions.

The cells kept under hyperglycaemia conditions, treated with the anti-diabetic drug show a considerable decrease in the response, in terms of photon emissions (lower than 40,000 cps) which means a good inhibition of the PKCβ-AEQ probe translocation.

In the control cells, no significant variation is observed of the emission of photons on the part of aequorin (lower than 10,000 cps).

From the graph, it appears evident that the treatment of cells kept under high glucose conditions by means of the anti-diabetic drug metformin, inhibits the translocation induced by glucose under hyperglycaemia conditions .

With this system, it is not only possible to identify the molecules capable of inhibiting a translocation, but also to evaluate its efficiency, by expressing it on the basis of a range between the cps value obtained under control conditions (0%)(without glucose) and that obtained following maximal translocation (100%) (after treatment with high glucose), which can be described as follows:
- GOOD inhibitor for values ranging from 10% to 40% with respect to the maximal activation;
- MEDIUM inhibitor for values ranging from 40% to 70% with respect to the maximal activation;
- POOR activator for values ranging from 70% to 100% with respect to the maximal activation.

This result clearly shows the validity of the system proposed for measuring the translocation of proteins from the cytosol compartment to the plasmatic membrane in response to external stimulations.

### EXAMPLE 3: Analysis of the translocation to the plasmatic membrane of the protein adaptor p66 belonging to the family of shc proteins.

### MATERIALS AND METHODS

### Construction of the chimeric p66shc-aequorin probe

A chimeric probe p66shc-aequorin was designed in order to further validate our procedure.

The nucleotide sequence of the protein p66shc used for the construction of the p66shc-aequorin probe has, as a reference: PUBMED 9049300.

The nucleotide sequence of aequorin used for the construction of p66shc-aequorin probes is the following:

ATG AAG CTT TAT GAT GTT CCT GAT TAT GCT AGC CTC AAA
CTT ACA TCA GAC TTC GAC AAC CCA AGA TGG ATT GGA CGA
CAC AAG CAT ATG TTC AAT TTC CTT GAT GTC AAC CAC AAT
GGA AAA ATC TCT CTT GAC GAG ATG GTC TAC AAG GCA TCT
GAT ATT GTC ATC AAT AAC CTT GGA GCA ACA CCT GAG CAA
GCC AAA CGA CAC AAA GAT GCT GTA GAA GCC TTC TTC GGA
GGA GCT GGA ATG AAA TAT GGT GTG GAA ACT GAT TGG CCT
GCA TAT ATT GAA GGA TGG AAA AAA TTG GCT ACT GAT GAA
TTG GAG AAA TAC GCC AAA AAC GAA CCA ACG CTC ATC CGT
ATA TGG GGT GAT GCT TTG TTT GAT ATC GTT GAC AAA GAT
CAA AAT GGA GCC ATT ACA CTG GAT GAA TGG AAA GCA TAC
ACC AAA GCT GCT GGT ATC ATC CAA TCA TCA GAA GAT TGC
GAG GAA ACA TTC AGA GTG TGC GAT ATT GAT GAA AGT GGA
CAA CTC GAT GTT GAT GAG ATG ACA AGA CAA CAT TTA GGA
TTT TGG TAC ACC ATG GAT CCT GCT TGC GAA AAG CTC TAC
GGT GGA GCT GTC CCC TAA.

This probe was obtained by inserting, in a eukaryotic expression vector (pcDNA3; 5.4 kb), the sequence encoding the p66shc protein condensed in frame with the sequence encoding aequorin.

The localization of the chimeric probe is determined by signal peptides contained inside the native sequence of the protein whose intracellular path is to be followed, in this specific case p66shc. The addition of the cDNA of aequorin to the C-terminal of the sequence encoding p66shc does not alter either the orientation or the physiological behaviour of the protein being examined.

At this point, transfection is effected of the chimeric protein probe obtained in different cellular lines, such as A431, DMS 79.

### Engineering of the cellular line

In the example illustrated, it was not necessary for the cellular line used to express any other element to activate the cellular response. It is possible, however, depending on study requirements, to induce the cells to express a specific receptor for the molecule/drug to be tested in order to ensure an effective linkage degree, thus simplifying the functional analysis of the transduction systems.

### Cellular transfection

The cells cultivated on cellular culture flasks (75 cm²) were transfected with a vector containing the chimeric probe produced, using the most suitable transfection techniques for the cellular line in question. The stabilized transfected cellular line A431 was adopted as experimental model, using the "Effectene reagent" procedure (Qiagen, Germany), which guarantees a high percentage of positive cells for this cellular line.

24 hours after the transfection, the cells were maintained in a DMEM culture medium, without serum.

### Collection of cells expressing the chimeric probe

36 hours after transfection, the cells contained in the flask were detached from the bottom by trypsinization. The cellular suspension was then transferred to a Falcon tube (15 to 50 ml) and subjected to centrifugation at 1200 rpm at 20°C and finally the cellular precipitate was re-suspended in KRB (Krebs-Ringer modified buffer: 125 mM NaCl, 5 mM KCl 1 mM Na₃PO₄, 1 mM MgSO₄ 5.5 mM glucose, 20 mM HEPES, pH 7.4, 37°C).

### Reconstitution of the aequorin photo-protein to active form

The prosthetic group celentherazine was added to the suspension of cells expressing the p66shc-aequorin probe, at a final concentration of 5 µM.

### Planting on multi-well plates

50 µl of suspension containing transfected cells (corresponding to about 100,000 cells) were planted in each well of the plate. The cells were left to adhere for a time varying from 1 to 2 hours keeping them in the dark, due to the photo-instability of celentherazine.

### Detection of the response

At the end of the incubation period, the various molecules to be tested were added to each well and the plate with the treated cells was then put in direct contact with a photo-multiplier which measures the emission of photons on the part of aequorin.

### RESULTS

This simple test can be used for testing compounds capable of modulating the translocation of proteins of the shc family in live cells.

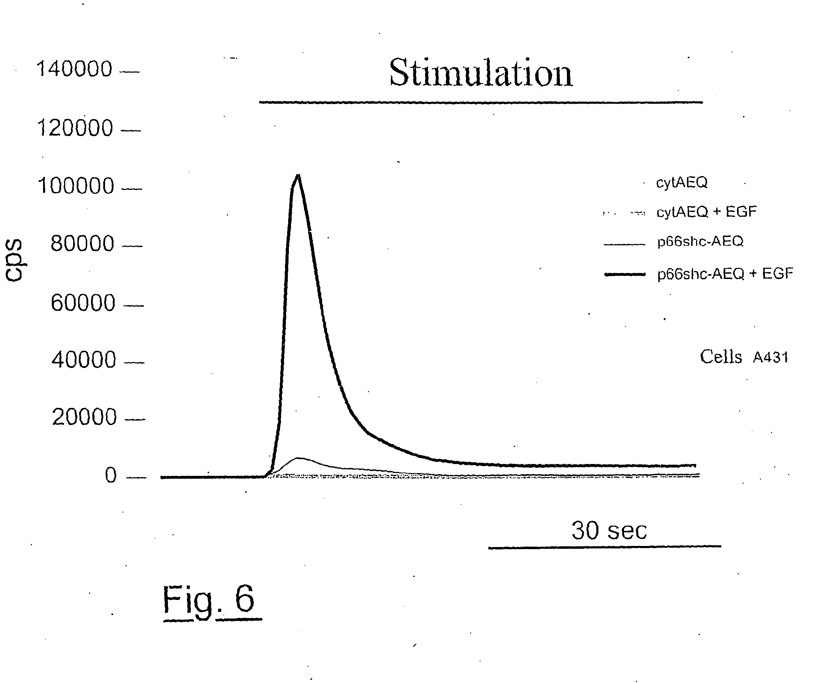

Figures 6 shows a graphs which indicates the measuring of the translocation of p66shc, according to the method described above.

The examples shown were carried out on two parallel batches of cells:
a) A431 cells expressing the p66shc-aequorin (p66shc-AEQ) probe (dark lines).
   These cells were in turn subdivided into:
   - A431 p66shc-aequorin cells treated with EGF (epidermic growth factor) (100 ng/ml) (thick dark line);
   - control A431 p66shc-aequorin cells, i.e. non treated with EGF (thin dark line);
b) A431 cells expressing the cytosol aequorin probe (cyt-AEQ) (not condensed to any protein) (light lines).

These cells were in turn subdivided into:
- A431 cells with cytosol aequorin treated with EGF (100 ng/mL) (thick light line);
- control A431 cells with cytosol aequorin, i.e. non treated with EGF (thin light line).

As can be observed from the trend of the graphs shown in figure 6, in the absence of external calcium, the emission of photons (expressed as cps: counts per second) has a very low intensity (lower than 100 cps), under all the conditions examined. Under such conditions, in fact, the concentration of calcium in the cytoplasm, where the cyt-AEQ probes (both in the absence and in the presence of EGF growth factor) and p66shc-AEQ probes (in the absence of EGF) are located, and below the plasmatic membrane, i.e. where the p66shc-AEQ probe translocates following treatment with EGF, is very low (0.1 µM).

The addition of external calcium induces a significant flow of this ion through the channels situated on the plasmatic membrane with a consequent increase in the concentration of calcium in the region below the plasmatic membrane. Under these conditions, an enormous increase in the emission of photons is registered (> 100,000 cps) only in the cells expressing the p66shc-AEQ probe, treated with EGF, indicating the complete translocation of the protein.

In all the other groups of cells, there is no significant variation in the emission of photons (lower than 10,000 cps) on the part of aequorin.

From the graph, it is evident that all the cps values higher than those observed in the absence of the maximal activator (EGF) will indicate a complete translocation of the kinase. With this system, it is therefore possible to identify, on the one hand, the molecules capable of inducing a translocation of the shc proteins through alternative paths with respect to that activated by the EGF growth factor, and, on the other hand, those molecules capable of blocking the intracellular transduction path activated by the EGF factor, by verifying the completed or non-completed translocation of the protein.

It is also possible to determine the efficiency with which a molecule induces (or blocks) said translocation by expressing it on the basis of a range between the cps value obtained under control conditions (0%) (without EGF) and that obtained following maximal translocation (100%) (after treatment with EGF), which can be described as follows:
- GOOD activator for values ranging from 70% to 100% with respect to the maximal activator;
- MEDIUM activator for values ranging from 40% to 70% with respect to the maximal activator;
- POOR activator for values ranging from 10% to 40% with respect to the maximal activator;
- NON-activator for values below 10% with respect to the maximal activator.
   This result clearly shows the validity of the system proposed for measuring the translocation of proteins from the cytosol compartment to the plasmatic membrane in response to external stimulations.

### BIBLIOGRAPHY

- Brini,M., M. Murgia, L.Pasti, D.Picard, T.Pozzan, and R.Rizzuto. 1993. EMBO J. 12:4813-4819.
- Brini,M., R.Marsault, C.Bastianutto, T.Pozzan, and R.Rizzuto. 1994a. Cell Calcium 16:259-268.
- Brini,M., L.Pasti, C.Bastianutto, M.Murgia, T.Pozzan, and R.Rizzuto. 1994b. J. Biolumin. Chemilumin. 9:177-184.
- Brini,M., R.Marsault, C.Bastianutto, J.Alvarez, T.Pozzan, and R.Rizzuto. '1995. J. Biol. Chem. 270:9896-9903.
- Brini,M., F.De Giorgi, M.Murgia, R.Marsault, M.L.Massimino, M.Cantini, R.Rizzuto and T.Pozzan. 1997. Mol. Biol. Cell 8:129-143.
- Brini,M., P.Pinton, T.Pozzan, and R.Rizzuto. 1999. Microsc.Res.Tech. 46:380-389.
- Chiesa,A., E.Rapizzi, V.Tosello, P.Pinton, M.de Virgilio, K.E.Fogarty; and R.Rizzuto. 2001. Biochem. J. 355:1-12.
- Cotecchia,S., F.Ostrowski, M.A.Kjelsberg, M.G.Caron, and R.J.Lefkowitz. 1992. J. Biol. Chem. 267:1633-1639.
- De Giorgi,F., M.Brini, C.Bastianutto, R.Marsault, M.Montero, P.Pizzo, and R.Rizzuto. 1996. Gene 173:113-117.
- Grynkiewicz.G., M.Poenie, and R.Y.Tsien. 1985. J. Biol. Chem. 260:3440-3450.
- Marsault,R. M.Murgia, T.Pozzan, and R.Rizzuto. 1997. EMBO J. 16:1575-1581.
- Montero,M., M.Brini, R.Marsault, J.Alvarez, R.Sitia, T.Pozzan, and R.Rizzuto. 1995. EMBO J. 14:5467-5475.
- Pinton,P., M.Brini, C.Bastianutto, R.A.Tuft, T.Pozzan, and R.Rizzuto. 1998. Biofactors 8:243-253.
- Pinton,P., T.Pozzan, and R.Rizzuto. 1998. EMBO J. 17:5298-5308.
- Porcelli,A.M., P.Pinton, E.K.Ainscow,, A.Chiesa, M.Rugolo, G.A.Rutter, and R.Rizzuto. 2001. Methods Cell Biol. 65:353-380.
- Ridgway,E.B. and C.C.Ashley. 1967. Biochem. Biophys. Res. Commun. 29:229-234.
- Rizzuto,R., A.W.Simpson, M.Brini, and T.Pozzan. 1992. Nature 358:325-327.
- Rizzuto,R., M.Brini, and T.Pozzan. 1993. Cytotechnology 11 Suppl 1:S44-S46.
- Rizzuto,R., M.Brini, and T.Pozzan. 1994. Methods Cell Biol. 40:339-358.
- Rizzuto,R., M.Brini, C.Bastianutto, R.Marsault, and T.Pozzan. 1995. Methods Enzymol. 260:417-428.
- Rizzuto,R., P.Pinton, W.Carrington, F.S.Fay, K.E.Fogarty, L.M.Lifshitz, R.A.Tuft, and T.Pozzan. 1998. Science 280:1763-1766.
- Robert,V., P.Pinton, V.Tosello, R.Rizzuto, and T.Pozzan. 2000. Methods Enzymol. 327:440-456.
- Rutter,G.A., P.Burnett, R.Rizzuto, M.Brini, M.Murgia, T.Pozzan, J.M.Tavare, and R.M.Denton. 1996. Proc. Natl. Acad. Sci. U.S.A. 93:5489-5494.
- Shimomura,O., F.H.JOHNSON, and Y.SAIGA. 1962. J. Cell Comp Physiol 59:223-239.
- Tsien,R.Y., T.Pozzan, and T.J.Rink. 1982. Nature 295:68-71.
- Zhang,J., R.E.Campbell, A.Y.Ting, and R.Y.Tsien. 2002. Nat. Rev. Mol. Cell Biol. 3:906-918
- Oancea,E., M.N.Teruel, A.F.G.Quest, and T.Meyer. 1998. J. Cell. Biol. 140: 485-489.

## Claims

1. Screening method of molecules capable of generating the alteration of a target intracellular parameter, said alteration being the translocation from cytoplasm to the membrane of a cellular effector, said translocation being correlated to the different intracellular concentration of the Ca²⁺ ion between cytoplasm and submembrane area, detected by means of a Ca²⁺-sensitive recombinant aequorin probe, comprising the following phases:
a) construction of an expression vector containing the fusion protein sequence encoding said probe, said sequence being **characterized in that** it comprises sequences encoding at least one Ca²⁺-sensitive recombinant aequorin encoding sequence, condensed together with at least one cellular effector portion coupled to translocation; wherein said cellular effector portion is selected between protein-kinases and adaptors;
b) transfection of at least one mammalian cell line with said vector containing the Ca²⁺-sensitive recombinant protein probe;
c) activation of said Ca²⁺-sensitive photo-protein by the addition of a prosthetic group to the cellular line expressing said recombinant protein probe;
d) administration of the molecule to be tested to the cellular line expressing said recombinant protein probe;
e) detection of the emission of photons on the part of the Ca²⁺-sensitive photo-protein expressed in the cellular line and evaluate the amount of activation or inhibition exerted by the tested molecule, on the basis of a ratio between the cps value obtained and the maximum value of cps registered under conditions of maximum stimulation of the cellular line.

2. Screening method according to claim 1, wherein said prosthetic group is celentherazine.

3. Screening method according to anyone of the claims 1 to 2, wherein said protein-kinase is protein-kinase C (PKC).

4. Screening method according to anyone of the claims 1 to 2, wherein said adaptor belongs to the she family.

5. Screening method according to claim 3 or 4, wherein said probe is a fusion protein selected from the group that consists of PKC-aequorin (PKC-AEQ) and shc-aequorin (shc-AEQ).

6. Screening method according to claim 5, wherein said PKC-aequorin is selected from the group comprising PKC beta-aequorin, PKC delta-aequorin, PKC epsilon-aequorin, PKC zeta-aequorin, PKC gamma-aequorin, PKC alpha-aequorin, PKC-lambda-aequorin, PKC theta-aequorin, PKC eta-aequorin.

7. Screening method according to claim 5, wherein the shc-aequorin is selected from the group consisting of p66shc-aequorin, p46shc-aequorin, p52shc-aequorin.

8. Screening method according to anyone of the claims 1 to 7, wherein the expression vector of phase a) is an eukaryotic vector.

9. Screening method according to anyone of the claims 1 to 8, wherein said at least one mammal cellular line of phase b) is previously engineered so as to express a heterologous native or chimeric protein.

10. Screening method according to claim 9, wherein said heterologous protein is selected from the group which consists of a receptor, an enzyme, a ionic channel and a cellular effector.

11. Screening method according to claim 9, wherein said chimeric protein is a chimeric receptor.

12. Screening method according to claim 10, wherein said ionic channel is selected from the group which comprises voltage-dependent Ca²⁺' channels and Ca²⁺ channel receptors.

13. Screening method according to claim 10, wherein said cellular effector is a regulating protein selected from the group which comprises protein-kinase, phosphatase, adenylate cyclase, proteins that links plasmatic membrane receptors, proteins that interacts with plasmatic membrane channels, proteins that interacts with plasmatic membrane lipids.

14. Screening method according to claim 10, wherein said cellular effector is a cell membrane receptor selected from the group which comprises receptors coupled with G proteins, receptors with an enzymatic activity, channel receptors.

## Patentansprüche

1. Verfahren zum Screening hinsichtlich von Molekülen, welche in der Lage sind, die Änderung eines intrazellulären Zielparameters zu verursachen, wobei die Änderung die Translokation eines zellulären Effektors vom Zytoplasma zur Membran ist, und wobei die Translokation mit der unterschiedlichen intrazellulären Konzentration des Ca²⁺-Ions zwischen Zytoplasma und Submembranregion korreliert ist, nachgewiesen mit Hilfe einer Ca²⁺-sensitiven rekombinanten Aequorin-Sonde, welches Verfahren die folgenden Phasen umfasst:
a) Konstruktion eines Expressionsvektors, der die Fusionsproteinsequenz enthält, welche für die Sonde codiert, wobei die Sequenz **dadurch gekennzeichnet ist, dass** sie Sequenzen umfasst, welche für mindestens eine für ein Ca²⁺-sensitives rekombinantes Aequorin codierende Sequenz codieren, fusioniert mit mindestens einem Anteil eines zellulären Effektors, der zur Translokation angekoppelt ist; wobei der Anteil des zellulären Effektors zwischen Proteinkinasen und Adaptoren ausgewählt ist;
b) Transfektion von mindestens einer Säugerzelllinie mit dem Vektor, welcher die Ca²⁺-sensitive rekombinante Protein-Sonde enthält;
c) Aktivierung des Ca²⁺-sensitiven Photoproteins durch Zugabe einer prosthetischen Gruppe zu der Zelllinie, welche die rekombinante Protein-Sonde exprimiert;
d) Verabreichung des zu testenden Moleküls an die Zelllinie, welche die rekombinante Protein-Sonde exprimiert;
e) Nachweis der Emission von Photonen von dem Ca²⁺-sensitiven Photoprotein, das in der Zelllinie exprimiert wird, und Evaluation des Ausmaßes an Aktivierung oder Inhibierung, welches von dem getesteten Molekül entfaltet wird, auf Basis eines Verhältnisses zwischen dem erhaltenen Cps-Wert und dem maximalen Cps-Wert, welcher unter Bedingungen maximaler Stimulation der Zelllinie registriert wird.

2. Screening-Verfahren nach Anspruch 1, wobei die prosthetische Gruppe Celentherazin ist.

3. Screening-Verfahren nach irgendeinem der Ansprüche 1 bis 2, wobei die Proteinkinase Proteinkinase C (PKC) ist.

4. Screening-Verfahren nach irgendeinem der Ansprüche 1 bis 2, wobei der Adaptor zu der shc-Familie gehört.

5. Screening-Verfahren nach Anspruch 3 oder 4, wobei die Sonde ein Fusionsprotein ist, ausgewählt aus der Gruppe, welche aus PKC-Aequorin (PKC-AEQ) und shc-Aequorin (shc-AEQ) besteht.

6. Screening-Verfahren nach Anspruch 5, wobei das PKC-Aequorin ausgewählt ist aus der Gruppe, welche PKC-Beta-Aequorin, PKC-Delta-Aequorin, PKC-Epsilon-Aequorin, PKC-Zeta-Aequorin, PKC-Gamma-Aequorin, PKC-Alpha-Aequorin, PKC-Lambda-Aequorin, PKC-Theta-Aequorin und PKC-Eta-Aequorin umfasst.

7. Screening-Verfahren nach Anspruch 5, wobei das shc-Aequorin ausgewählt ist aus der Gruppe, welche aus p66shc-Aequorin, p46shc-Aequorin und p52shc-Aequorin besteht.

8. Screening-Verfahren nach irgendeinem der Ansprüche 1 bis 7, wobei der Expressionsvektor von Phase a) ein eukaryotischer Vektor ist.

9. Screening-Verfahren nach irgendeinem der Ansprüche 1 bis 8, wobei die mindestens eine Säugerzelllinie von Phase b) zuvor gentechnisch manipuliert wurde, um ein heterologes natives oder chimäres Protein zu exprimieren.

10. Screening-Verfahren nach Anspruch 9, wobei das heterologe Protein aus der Gruppe ausgewählt ist, welche aus einem Rezeptor, einem Enzym, einem Ionenkanal und einem zellulären Effektor besteht.

11. Screening-Verfahren nach Anspruch 9, wobei das chimäre Protein ein chimärer Rezeptor ist.

12. Screening-Verfahren nach Anspruch 10, wobei der Ionenkanal aus der Gruppe ausgewählt ist, welche spannungsabhängige Ca²⁺-Kanäle und Ca²⁺-Kanal-Rezeptoren umfasst.

13. Screening-Verfahren nach Anspruch 10, wobei der zelluläre Effektor ein Regulationsprotein ist, ausgewählt aus der Gruppe, welche Proteinkinase, Phosphatase, Adenylatcyclase, Proteine, die Plasmamembranrezeptoren verknüpfen, Proteine, die mit Plasmamembrankanälen interagieren, und Proteine, die mit Plasmamembranlipiden interagieren, umfasst.

14. Screening-Verfahren nach Anspruch 10, wobei der zelluläre Effektor ein Zellmembranrezeptor ist, ausgewählt aus der Gruppe, welche mit G-Proteinen gekoppelte Rezeptoren, Rezeptoren mit einer enzymatischen Aktivität und Kanal-Rezeptoren umfasst.

## Revendications

1. Procédé de criblage de molécules capables de produire une altération d'un paramètre intracellulaire cible, ladite altération étant la translocation du cytoplasme vers la membrane d'un effecteur cellulaire, ladite translocation étant corrélée avec la différence de concentration intracellulaire de l'ion Ca²⁺ entre le cytoplasme et la région sous-membranaire, détectée à l'aide d'une sonde aequorine recombinante sensible à Ca²⁺, comprenant les phases suivantes:
a) construction d'un vecteur d'expression contenant la séquence de protéine de fusion codant pour ladite sonde, ladite séquence étant **caractérisée en ce qu'**elle comprend des séquences codant pour au moins une séquence codante d'aequorine recombinante sensible à Ca²⁺, condensées avec au moins une portion d'effecteur cellulaire couplée à la translocation; ladite portion d'effecteur cellulaire étant choisie entre des protéines-kinases et des adaptateurs;
b) transfection d'au moins une lignée cellulaire de mammifère avec ledit vecteur contenant la sonde protéique recombinante sensible à Ca²⁺;
c) activation de ladite photo-protéine sensible à Ca²⁺ grâce à l'addition d'un groupe prosthétique à la lignée cellulaire exprimant ladite sonde protéique recombinante;
d) administration de la molécule à tester à la lignée cellulaire exprimant ladite sonde protéique recombinante;
e) détection de l'émission de photons sur la partie de la photo-protéine sensible à Ca²⁺, exprimée dans la lignée cellulaire et évaluation du niveau d'activation ou d'inhibition exercée par la molécule testée, sur la base d'un rapport entre les valeurs de cp obtenues et la valeur de cp maximale enregistrée dans des conditions de stimulation maximale de la lignée cellulaire.

2. Procédé de criblage selon la revendication 1, dans lequel ledit groupe prosthétique est la célenthérazine.

3. Procédé de criblage selon l'une quelconque des revendications 1 à 2, dans lequel ladite protéine-kinase est la protéine-kinase C (PKC).

4. Procédé de criblage selon l'une quelconque des revendications 1 à 2, dans lequel ledit adaptateur appartient à la famille shc.

5. Procédé de criblage selon la revendication 3 ou 4, dans lequel ladite sonde est une protéine de fusion choisie dans le groupe constitué par la PKC-aequorine (PKC-AEQ) et la shc-aequorine (shc-AEQ).

6. Procédé de criblage selon la revendication 5, dans lequel ladite PKC-aequorine est choisie dans le groupe comprenant la PKC bêta-aequorine, la PKC delta-aequorine, la PKC epsilon-aequorine, la PKC zêta-aéquorine, la PKC gamma-aequorine, la PKC alpha-aequorine, la PKC lambda-aequorine, la PKC thêta-aequorine, la PKC êta-aequorine.

7. Procédé de criblage selon la revendication 5, dans lequel la shc-aequorine est choisie dans le groupe constitué par la p66shc-aequorine, la p46shc-aequorine, la p52shc-aequorine.

8. Procédé de criblage selon l'une quelconque des revendications 1 à 7, dans lequel le vecteur d'expression de la phase a) est un vecteur eucaryote.

9. Procédé de criblage selon l'une quelconque des revendications 1 à 8, dans lequel ladite au moins une lignée cellulaire de mammifère de la phase b) est préalablement manipulée génétiquement pour exprimer une protéine chimère ou native hétérologue.

10. Procédé de criblage selon la revendication 9, dans lequel ladite protéine hétérologue est choisie dans le groupe constitué par un récepteur, une enzyme, un canal ionique et un effecteur cellulaire.

11. Procédé de criblage selon la revendication 9, dans lequel ladite protéine chimère est un récepteur chimère.

12. Procédé de criblage selon la revendication 9, dans lequel ledit canal ionique est choisi dans le groupe qui comprend des canaux de Ca²⁺ potentiel-dépendants et des récepteurs de canaux de Ca²⁺.

13. Procédé de criblage selon la revendication 10, dans lequel ledit effecteur cellulaire est une protéine régulatrice choisie dans le groupe qui comprend une protéine-kinase, une phosphatase, une adénylate-cyclase, des protéines qui se lient aux récepteurs de la membrane plasmatique, des protéines qui interagissent avec les canaux de la membrane plasmatique, des protéines qui interagissent avec les lipides de la membrane plasmatique.

14. Procédé de criblage selon la revendication 10, dans lequel ledit effecteur cellulaire est un récepteur de membrane cellulaire choisi dans le groupe qui comprend des récepteurs couplés aux protéines G, des récepteurs ayant une activité enzymatique , des récepteurs de canaux.
